# EUROPEAN PATENT SPECIFICATION

(11) **EP 3 262 200 B1**
(45) Date of publication and mention of the grant of the patent: **27.03.2019**
(21) Application number: 16705963.3
(22) Date of filing: 24.02.2016
(51) Int. Cl.: C13K 5/00, C07H 3/04, A61K 9/20, A23C 21/00

(54) **PROCESS FOR PRODUCING LOW ASH LACTOSE USING A CLARIFIER WITH ACID AND DEVICE FOR CARRYING OUT THE PROCESS**
VERFAHREN ZUR HERSTELLUNG VON LACTOSE MIT NIEDRIGEM ASCHEGEHALT MITTELS EINER KLÄRUNGSVORRICHTUNG MIT SÄURE SOWIE VORRICHTUNG ZUR DURCHFÜHRUNG DES VERFAHRENS
PROCÉDÉ DE PRODUCTION DE LACTOSE À FAIBLE TENEUR EN CENDRES AU MOYEN D'UN CLARIFICATEUR AVEC DE L'ACIDE ET DISPOSITIF POUR LA MISE EN OEUVRE DU PROCÉDÉ

(30) Priority: 28.02.2015 DK 201500115; 10.07.2015 DK 201500409; 04.09.2015 DK 201500528
(43) Date of publication of application: 03.01.2018
(73) Proprietor: Spx Flow Technology Danmark A/S, 2860 Søborg (DK)
(72) Inventor: WAGNER, Peter, DK-2970 Hørsholm (DK); DALBERG, Anders, DK-7451 Sunds (DK); POULSEN, Olav, DK-8870 Langå (DK)
(74) Representative: Nordic Patent Service A/S
(86) International application number: PCT/EP2016/053821
(87) International publication number: WO 2016/135172

(56) References cited:
- EP-A1- 2 617 727
- EP-A2- 0 311 977
- WO-A1-96/23417
- GB-A- 200 185
- GB-A- 1 575 089
- US-A- 4 099 983
- US-A- 4 342 604
- GEA Mechanical Equipment: "Prozesslinien von GEA Westfalia Separator zur Verarbeitung von Molke", Internet , 2013, pages 1-33, XP002757092, Retrieved from the Internet: URL:https://www.yumpu.com/de/document/view /7949755/prozesslinien-von-gea-westfalia-s eparator-zur-verarbeitung-von- [retrieved on 2016-04-26]
- GEA Mechanical Equipment: "Process Stages, Systems and Equipment for the Dairy Industry from GEA Westfalia Separator", Internet , pages 1-12, XP002757093, Retrieved from the Internet: URL:http://www.novacro.com.ar/archivos/240 _1380400707_dairy-industry-process-stages- systems-equipment-9997-1390-010.pdf [retrieved on 2016-04-26]

## Description

### FIELD

The invention relates to a method of combined calcium precipitation, separation by use of centrifugal clarifier and pH adjustment prior to further processing in the production of lactose from whey permeate with very high purity and very low ash levels after wash.

### BACKGROUND

In the production of crystalline lactose from whey or whey permeate the presence of salts in the whey, which may co-precipitate with lactose during lactose crystallization, is a persisting problem. The concentration of mineral salts in the whey is typically from 5-40 mM with calcium salts, in particular calcium phosphates, being particularly problematic for the efficient production of lactose from whey permeate due to calcium's high content in milk and the atypical precipitation behavior of calcium phosphates, which exhibit reduced solubility with increased temperature.
In the industry it is common to concentrate whey permeate to higher solids content using evaporation prior to the precipitation of lactose, see e.g. WO 2012/047122. However when evaporating and if calcium phosphates are not inactivated prior to the whey permeate entering the evaporator, the salts may precipitate on the heat surfaces in the evaporator causing reduced heating efficacy and additional downtime for maintenance and cleaning.

Traditionally, calcium phosphate is precipitated in the permeate by adjusting the pH to about 7.2, e.g. by addition of caustic Mg(OH)₂ or NaOH, after which the permeate is heated to about 80°C. The precipitated calcium phosphate can then be removed by centrifugation or membrane filtration. Usually, the discharged product is then dried and sold as food additives ("milk calcium") or used in the manufacture of fertilizers, e.g. in the Odda-process.

However, with the traditional industrial methods of precipitating calcium phosphates from whey it is difficult to obtain consistent ash levels below 0.3% in the resulting lactose powders due to incorporated Ca and P in the formed lactose crystals. This ash level, however, is important as 0.3% ash content is the current maximum limit in lactose used for infant formulas. As a result thereof, lactose for infant formulas must currently undergo additional processing to reduce the ash levels therein to meet current standards for infant formulas. This increases cost to the detriment of industry and families with infants that rely on infant formulas for the nutrition of their children.

Accordingly, there is a need in the art for industrial scale methods of precipitating calcium phosphates from whey permeate for obtaining crystallized lactose with low ash levels of calcium phosphates.

This problem has traditionally been solved by submitting whey to ultrafiltration and ion-exchange at high temperatures, thereby obtaining whey permeate with low ash levels of calcium phosphates. However this method is generally considered expensive in establishing and operating costs.

US 4,202,909 describes methods of precipitating calcium salts from whey at the pH of milk, while leaving phosphate-ions in the whey permeate, involving precipitating by heating and removing the resulting precipitate, primarily calcium citrate salts, by clarifying.
An alternative method exists which combines submitting the whey to ultrafiltration to obtain a permeate enriched in calcium and phosphate, followed by calcium precipitation at elevated temperature and basic pH, followed by cooling, and separation of the calcium precipitate by use of a clarifier to remove precipitated calcium phosphates and obtain a whey permeate with reduced ash levels of calcium phosphates of 0.2%, see e.g. http://us.westfalia-separator.com/fileadmin/GEA WS US/Documents/Brochures/ Dairy/Processing Lines for Whey Brochure.pdf; accessed February 26, 2015 or https://www.yumpu.com/de/document/ view/7949755/prozesslinien-von-gea-westfalia-separator-zur-verarbeitung-von- ;published 2013, XP002757092. In this process, calcium phosphate is separated from whey permeate. The purpose of this method is to obtain calcium phosphate as a food additive and also to achieve longer operating times in the concentration processes due to the effect of demineralizing the permeate.

EP261772727 discloses a process for yield improvement in the recovery of essentially mineral-free lactose. The process comprises subjecting whey to a separation process; optionally concentration the obtained lactose rich fraction to main stream and subjecting to demineralization; cooling the demineralized residue; and separating the lactose crystals from the mother liquor, and dehydrating to obtain a first amount of lactose, where the mother liquor separated from the main stream is subjected to e.g. demineralization, cooling, separation of crystals and drying process and mixed with the first amount.

The current inventors have now realized that by modifying the above alternative process, an improved process for the separation of calcium phosphates from whey permeate is obtained which in one step can yield ash levels below 0.2% and as low as 0.1% without the need for further purification of the whey permeate. Further, improvements in energy savings are thereby achieved, e.g. as in some embodiments processing steps of cooling and subsequent re-heating can be dispensed with. The process of the present invention is particularly suitable in the dairy industry where large quantities of whey permeate are handled and where improvements to process economy is of particular importance. A further advantage of the presently suggested process is increased versatility of a production line implementing the process, permitting the line to produce both high grade and low grade lactose using the same equipment, thereby reducing CAPEX and OPEX for the production line.

### SUMMARY OF THE INVENTION

In a first aspect and embodiment, the present invention concerns a process for producing lactose from concentrated whey permeate comprising between 10 to 70% dry solids, said dry solids comprising calcium phosphate, said process comprising the steps of:
ii. Forming precipitates of calcium phosphate in said whey permeate in a precipitation step (5), a fraction of said precipitates having a size larger than a centrifugal clarifier cut-off value;
iii. Clarifying the whey permeate of step ii. in a centrifugal clarification step (7) to remove said fraction of said precipitates having a size larger than said centrifugal clarifier cut-off value to obtain a calcium phosphates reduced whey permeate;
iv. Adding an acid to the calcium phosphates reduced whey permeate of step iii. in an acid addition step (8) to dissolve any remaining precipitates of calcium phosphate, thereby obtaining a precipitate free whey permeate;
vi. Precipitating lactose from said precipitate free whey permeate of step iv. in a lactose precipitation step (10).

In a second embodiment of the process according to the first embodiment, the process further comprising the step of:
i. Independently, adjusting the pH of the concentrated whey permeate to between pH 6.0 to pH 8.5 in a pH-adjustment step (3) and/or heating the whey permeate to between 60°C to 90°C in a heating step (4) prior to forming said precipitates in step ii.

In a third embodiment of the process according to the first or second embodiments, the process further comprising the step of:
v. Concentrating the precipitate free whey permeate of step iv. in a lactose concentration step (9) to obtain a high lactose content whey permeate prior to precipitating lactose in said lactose precipitation step (10).

In a fourth embodiment of the process according to any of the first to third embodiments wherein, when step v. is present, said concentrated whey permeate preferably comprises between 10 to 40% dry solids, most preferably 20% dry solids, and when step v. is absent, said concentrated whey permeate preferably comprises between 45 to 70% dry solids, most preferably 60% dry solids.

In a fifth embodiment of the process according to any of the first to fourth embodiments wherein when step i. is performed, said precipitation step (5) is executed for at least 30 minutes.

In a sixth embodiment of the process according to any of the first to fifth embodiments, said concentrated whey permeate comprising between 10 to 70% dry solids, preferably 10 to 40% dry solids, said process comprising the steps of:
i. Independently, adjusting the pH of the concentrated whey permeate to between pH 6.0 to pH 8.5 in a pH-adjustment step (3) and/or heating the whey permeate to between 60°C to 90°C in a heating step (4);
ii. Holding the concentrated whey permeate of step i. for a retention time of at least 30 minutes in a precipitation step (5) in order to allow precipitates of calcium phosphates having a size larger than a clarifier cut-off value to form in the whey permeate;
iii. Clarifying the whey permeate of step ii. in a centrifugal clarification step (7) to remove precipitates of calcium phosphates with sizes above said centrifugal clarifier cut-off value to obtain a calcium phosphates reduced whey permeate;
iv. Adding acid to the calcium phosphates reduced whey permeate of step iii. in an acid addition step (8) to dissolve precipitates of calcium phosphate smaller than said clarifier cut-off value, thereby obtaining a precipitate free whey permeate;
v. Concentrating the precipitate free whey permeate of step iv. in a lactose concentration step (9) to obtain a high lactose content whey permeate; and
vi. Precipitating lactose from said high lactose content whey permeate of step v. in a lactose precipitation step (10).
In a seventh embodiment of the invention, according to any of the second to sixth embodiments comprising both a pH-adjustment step (3) and a heating step (4).

In an eight embodiment of the process according to any of said first to seventh embodiment wherein step ii. or, when present, said step i. is preceded by a permeate concentration step (2) and optionally, a riboflavin removal step.
In a ninth embodiment of the process according to any of previous embodiments, the process comprises addition of a base to maintain the pH during said precipitation step (5) at the pH of the pH-adjustment step (3).
In a tenth embodiment of the process according to any of the previous embodiments said centrifugal clarifier cut-off value is 1 µm.
In an eleventh embodiment of the process according to any of the previous embodiments the process comprises adjusting pH down by 0.05 to 0.5 pH degrees in said acid addition step (8).
In a twelfth embodiment of the process according to any of the third to eleventh embodiments the process comprises said lactose concentration step (9) being concentration by evaporation.
In a thirteenth embodiment of the process according to any of the previous embodiments wherein in the precipitation step (5) a base is added to maintain pH at a constant level. In a fourteenth embodiment there is disclosed a lactose line (20) configured for producing lactose from a source of concentrated whey permeate comprising between 10 to 40% dry solids (30), said lactose line (20) comprising: a permeate flow path (28) configured for permitting whey permeate to traverse said lactose line (20); a sequence of process units (21,22,23,24,25,26a,26b,27) operatively arranged on said permeate flow path (28), in a first section comprising a heating unit (21), a first caustic tank (26a) configured for addition of a base to said whey permeate, and optionally a permeate bypass flow path (29) for bypassing said heating unit; a second section comprising a holding tank (22), a centrifugal clarifier (23), and optionally a second caustic tank (26b) configured for addition of a base to said holding tank; a third section comprising an acid tank (27) and a concentration unit (24); and a fourth section comprising a lactose precipitation unit (25); at least one pump for pumping concentrated whey permeate through said lactose line (20); and a controller configured for executing a process according to any of the first to thirteenth embodiments of the present invention; wherein in said first section a pH-adjustment step (3) and/or a heating step (4) can be executed; in said second section a precipitation step (5) can be executed in said holding tank (22), optionally under addition of base from said optional second caustic tank (26b), and followed by a clarification step (7); in said third section an acid addition step (8) can be executed followed by a lactose concentration step (9); and in said fourth section a lactose precipitation step (10) can be executed thereby yielding solid lactose (33) and mother liquor (34) depleted in lactose.

### BRIEF DESCRIPTION OF THE DRAWINGS

Figure 1: Flow diagrams of prior art process and exemplary processes for the separation of calcium phosphates from whey permeate according to the present invention.
Figure 2: Lactose line for executing a process according to the invention.

### DETAILED DESCRIPTION

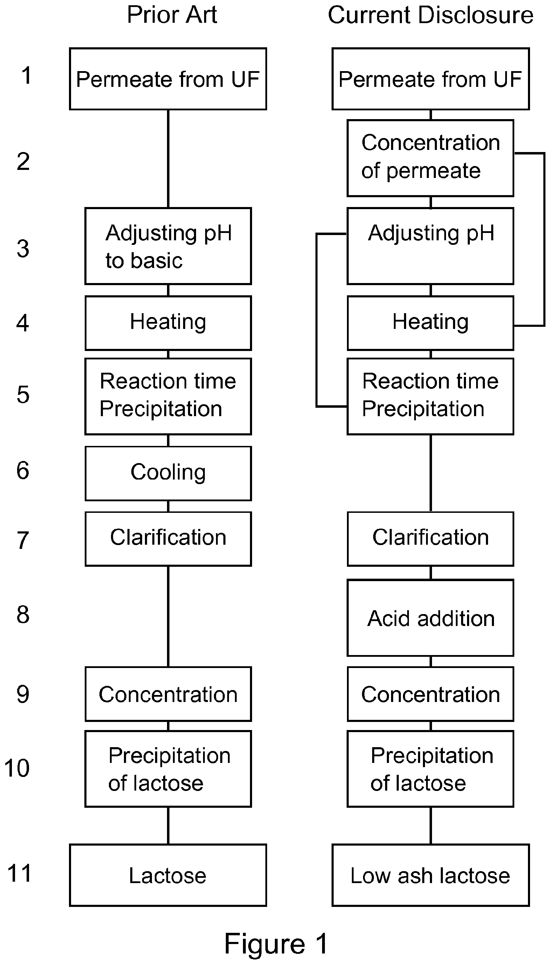

The improved process for the separation of calcium phosphates from whey permeate according to the present invention is described below with reference to the drawing, Figure 1. The processes described in the flow-diagram are meant as an illustration of the inventive concept, and the skilled person will be able to make modifications thereof, following the contents of the present disclosure, without departing from the disclosed inventive concept.
In general there is disclosed with the invention a process for the production of low ash levels lactose crystals comprising clarifying a whey permeate after calcium precipitation followed by pH-adjustment to dissolve precipitates of calcium phosphates not removed by clarification.
Accordingly there is disclosed herein a process for producing lactose from concentrated whey permeate comprising between 10 to 70% dry solids, the dry solids comprising calcium phosphate, the process comprising the steps of: ii. Forming precipitates of calcium phosphate in the whey permeate in a precipitation step (5), a fraction of the precipitates having a size larger than a centrifugal clarifier cut-off value; iii. Clarifying the whey permeate of step ii. in a clarification step (7) to remove the fraction of the precipitates having a size larger than the clarifier cut-off value to obtain a calcium phosphates reduced whey permeate; iv. Adding an acid to the calcium phosphates reduced whey permeate of step iii. in an acid addition step (8) to dissolve any remaining precipitates of calcium phosphate, thereby obtaining a precipitate free whey permeate; vi. Precipitating lactose from the precipitate free whey permeate of step iv. in a lactose precipitation step (10).
In a particularly preferred embodiment of the present invention there is disclosed a process as detailed below for producing lactose from concentrated whey permeate, said whey permeate comprising between 10 to 70% dry solids, preferably 10 to 40% dry solids, said process comprising the steps of: i. Independently, adjusting the pH of the concentrated whey permeate to between pH 6.0 to pH 8.5 in a pH-adjustment step (3) and/or heating the whey permeate to between 60°C to 90°C in a heating step (4); ii. Holding the concentrated whey permeate of step i. for a retention time of at least 30 minutes in a precipitation step (5) in order to allow precipitates of calcium phosphates having a size larger than a centrifugal clarifier cut-off value to form in the whey permeate; iii. Clarifying the whey permeate of step ii. in a clarification step (7) to remove precipitates of calcium phosphates with sizes above said clarifier cut-off value to obtain a calcium phosphates reduced whey permeate; iv. Adding an acid to the calcium phosphates reduced whey permeate of step iii. in an acid addition step (8) to dissolve precipitates of calcium phosphate smaller than said clarifier cut-off value, thereby obtaining a precipitate free whey permeate; v. Concentrating the precipitate free whey permeate of step iv. in a lactose concentration step (9) to obtain a high lactose content whey permeate; and vi. Precipitating lactose from said high lactose content whey permeate of step v. in a lactose precipitation step (10).

In the prior art it is known to form precipitates of calcium phosphate in whey permeate (cf. figure 1) by adjusting pH to basic (3), heating (4), precipitating calcium phosphate (5), followed by cooling (6) and removal of the formed precipitates by clarification (7). However, the resulting calcium reduced whey permeate is unsatisfactory for use in lactose production as not all of the precipitated calcium phosphate is removed by the clarification procedure. Further the process is inefficient from an energy perspective as it requires consecutive steps of both heating (4) and cooling (6) prior to clarification (7).

In particular, the partial removal of calcium phosphate constitutes a problem since calcium phosphate not removed by clarification has a high propensity to co-precipitate with lactose, when lactose is crystallized, and the present invention further aims at overcoming this problem.

The present inventors have surprisingly discovered that the problem of co-precipitation can be solved in a simple manner by re-dissolving formed calcium phosphate crystals not removed by clarification by acid addition to the resulting whey permeate after clarification. However, it is a welcome additional benefit of the present invention that when following the process according to the invention, heating is only optional and that, when the whey permeate is optionally heated, cooling can be dispensed with.

Below, the invention is described by way of example in accordance with a best mode of performing the process of the invention; however, deviations from this best mode comprised by the invention are detailed in the claims and the description.

Whey permeate comprising calcium phosphates dissolved in an aqueous phase is used as a starting material (1), usually whey permeate from ultrafiltration. It is normally contemplated that the whey permeate comprises calcium phosphates in concentrations equal to those found in the milk from which the whey originated, however, whey permeates comprising calcium phosphates in concentrations higher or lower than this are equally suitable for use with the process of the invention. The whey permeate will typically originate from ultrafiltration of whey, however, other methods of isolating whey proteins from whey permeate yield equally useful starting materials and hence, the present invention is not limited by the manner in which whey proteins are separated from whey permeate.

In accordance with the process of the invention as detailed in the exemplary flow-diagram of figure 1, whey permeate from a whey permeate source is concentrated to remove surplus water in a permeate concentration step (2). It is generally preferred that concentration is by reverse osmosis, but other methods of concentrating whey permeate are equally useful. Depending on the demands of the process, whey permeate can be concentrated to between 10 to 40% dry solids (DS), between 15 to 35% DS, between 17 to 30% DS, preferably between 18 to 25% DS, and most preferably 20% DS. In other processes whey permeate can be concentrated to between 45 to 70% DS, to between 50 to 65% DS, to between 55 to 60% DS, most preferably to 60% DS. In general, the process of the invention is suitable for use with concentrated whey permeate comprising between 10 to 70% DS.

The permeate concentration step (2) may be combined with a riboflavin removal step for the removal of riboflavin present in the whey permeate, e.g. by passing the concentrated whey permeate through a column comprising active carbon, thereby removing riboflavin by adsorption.

The permeate concentration step (2) is followed by adjusting the pH in a pH-adjustment step (3), and/or a heating step (4), and a precipitation step (5), preferably a precipitation step at an elevated temperature. As will be detailed below, the steps of adjusting pH (3) and of heating (4) can be used individually without the other or in combination, the combination being preferred.

Accordingly, the process of the invention may comprise the step i. of, independently, adjusting the pH of the concentrated whey permeate to between pH 6.0 to pH 8.5 in a pH-adjustment step (3) and/or heating the whey permeate to between 60°C to 90°C in a heating step (4) prior to forming said precipitates in step ii.

Whey permeate in general has a pH below 6.3. Once pH is raised above six (see e.g. US 6558717 B1) calcium phosphates can be expected to precipitate from solution. Already at pH 6.5 at least 50% precipitation can be expected and raising the pH to moderate basic conditions, such as pH 7.2, will cause more than 65% of the calcium phosphate to precipitate due to the influence of pH alone. Raising the pH to above 8 does not further benefit the precipitation of calcium phosphates. Accordingly, in the pH-adjustment step (3) as contemplated in the present invention, pH is adjusted to lie between at least 6.0 and not more than 8.5, between 6.1 and 8.0, between 6.2 and 7.8. More preferably, pH is adjusted to lie between 6.3 and 7.5, between 6.4 and 7.2, or between 6.5 and 6.9. Most preferably, pH is adjusted to be 6.5. The skilled person will know how to adjust to the pH for use in the present process, if further optimization is contemplated beyond what is stated here.

In general, due to the buffering capacity of the whey permeate and, in particular, the phosphates therein, pH adjustment requires equimolar amounts of base to buffer, such that one mole buffering capacity will require one mole base to neutralize. In this respect, the method of the invention differs significantly from the commonly applied methods of washing formed lactose crystals in slightly acidified or basic solutions to remove surface impurities from the formed crystals.

The heating step (4) involves heating the concentrated whey permeate to a temperature of precipitation above 60°C and below 90°C. Improved results are achieved when the temperature is between 65°C to 85°C, more preferably between 70°C to 80°C, more preferably between 73°C to 78°C, and most preferably around 75°C. These temperatures of precipitation are well known in the art and the skilled person will know how to adjust to the temperature for use in the present process, if further optimization is contemplated beyond what is stated here.

In order to achieve the beneficial high level removal of ash in crystallized lactose made from permeate treated using the presently suggested process, heating (4) and pH-adjustment (3) should be combined, however, due to the permeate concentration step (2), significant precipitation of calcium phosphates can be achieved with the process without the use of both pH-adjustment or heating, as long as at least one of these process steps is used. Thereby a lactose crystallization plant or production line (20) operating according to the present process gains additional versatility, as it may produce both low grade and high grade lactose without the need for dedicated low grade and high grade production lines. Also, and in general, omitting a process step reduces production costs, which may be crucial for competitive low grade products.

The pH-adjustment step (3) and/or heating step (4) is followed by a precipitation step (5) in which the concentrated permeate is retained in a precipitation vessel for a retention time sufficient to allow calcium phosphate crystals larger than at least 0.5 µm to form. An average retention time for precipitation is preferably from between 30 min to 120 min, most preferably around 60 minutes, as is known in the art. The skilled person will know how to optimize the retention time to the process economy desired. For high level ash removal retention times of 60 minutes or more are usually sufficient, when both the pH-adjustment step (3) and the heating step (4) have been implemented.

In one embodiment a base is added during the precipitation step (5) at the rate of calcium phosphate precipitation in order to maintain the pH constant, e.g. at the level obtained in the pH-adjustment step (3). Generally, as calcium phosphates in solution are slightly basic, the removal of calcium phosphates will drive the pH towards acidic, which is advantageously compensated for in the suggested embodiment.

The precipitation step (5) is followed by a clarification step (7), preferably at the temperature of the precipitation step (5). In the preferred embodiment, energy is saved as cooling or further heating can be omitted. Further, when operating the process for obtaining high-level ash removal, retaining the temperature of the clarification step (7) substantially at the temperature of the precipitation step (5) will help to drive the equilibrium towards precipitation as calcium phosphates can be continuously removed during precipitation, thereby further reducing the ash levels in lactose produced based on the presently suggested process.

Current clarifiers, such as the SPX Seital Separation Technology clarifier range, allow the recovery of fine particles above a given clarifier cut-off value, typically 0.5-500 µm. Hence retention times in the precipitation step must be sufficient to obtain calcium phosphate crystals of at least a clarifier cut-off value of 0.5 µm. However, in the present process, it is preferable that the retention time is sufficient to generate crystals of a clarifier cut-off value of at least 1 µm for optimal efficacy of the clarifier employed. Other means of centrifugal removal of precipitates as known to the skilled person, such as e.g. centrifuges, may also be used.

Clarification in step (7) is followed by an acid addition step (8) wherein the calcium phosphates reduced permeate is pH-adjusted down by 0.05 to 0.5 pH degrees. The pH may, depending on the needs in the further downstream processes, in some instances be reduced further. By reducing the pH of the permeate, precipitates of calcium phosphates which were too small to be removed in the clarification step (7) will be dissolved. Due to the removal of phosphate from the permeate during precipitation, the pH changes and the buffering capacity of the permeate reduces. Accordingly, it can be beneficial to add base continuously during precipitation, if the pH-adjustment step (3) option is used. Alternatively, the pH drops during precipitation. Since the mass of the very fine calcium phosphates particles in the permeate is very low, and the buffer capacity is reduced by the significant removal of phosphate, the amount of acid required for neutralization and dissolution is relatively small, albeit equimolar amounts will still be needed for full dissolution.

The permeate, which has now become a calcium phosphates reduced permeate, may subsequently be submitted to further concentration, in order to concentrate the lactose comprised therein in a lactose concentration step (9) prior to precipitation of lactose in a lactose precipitation step (10) to yield crystalline lactose (11). Methods of concentration and precipitation of lactose as generally known in the art are all suitable for these subsequent steps.

Accordingly, the process of the invention may comprise in a step v. concentrating the precipitate free whey permeate of step iv. in a lactose concentration step (9) to obtain a high lactose content whey permeate prior to precipitating lactose in said lactose precipitation step (10). When step v. is present, said concentrated whey permeate preferably comprises between 10 to 45 % dry solids, most preferably 20 % dry solids, and when step v. is absent, said concentrated whey permeate preferably comprises between 45 to 70 % dry solids, most preferably 60 % dry solids as detailed above in relation to the permeate concentration step (2).

A particularly suitable lactose concentration step (9) is by evaporation according to known methods in the art. When lactose concentration is performed by evaporation of the calcium phosphates reduced permeate, it is an additional advantage of the presently suggested process that when a heating step (4) is employed prior to and during calcium phosphates precipitation (5) and clarification (7), the permeate reaching the concentration step (9) is already heated to the precipitation temperature and less or no heating is needed to get the desired inlet temperature of the evaporator.

The below examples show the effect of producing crystalline lactose according to the presently suggested process following the preferred process of pH-adjustment and heating to obtain low ash level lactose crystals.

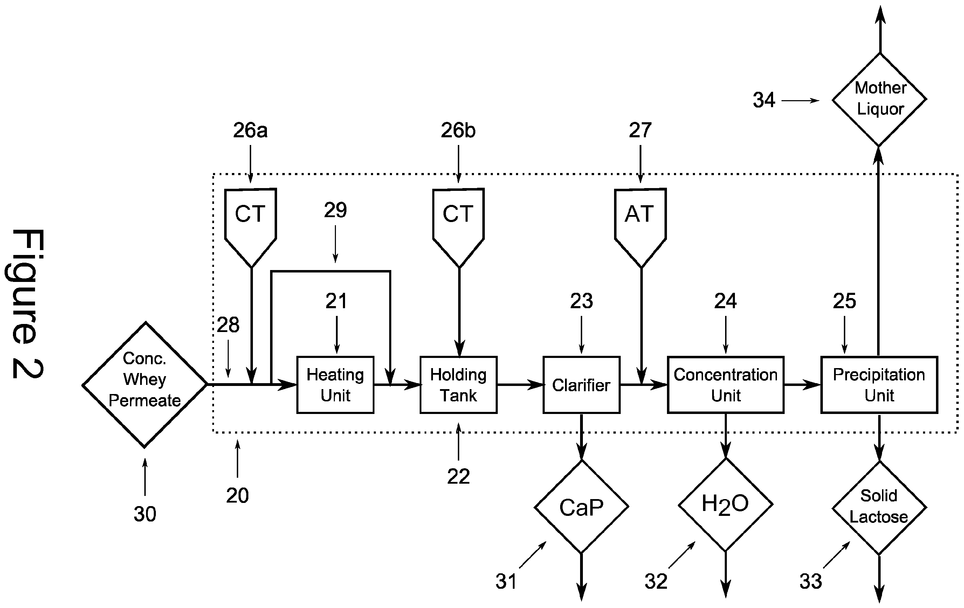

In a further aspect of the invention there is disclosed an arrangement, e.g. a production line herein called a lactose line (20) configured for executing a process according to any of the above embodiments. This embodiment is detailed further below with reference to Figure 2.

Accordingly there is disclosed in one embodiment a lactose line (20) configured for producing lactose from a source of concentrated whey permeate comprising between 10 to 70% dry solids (30), said lactose line (20) comprising: a permeate flow path (28) configured for permitting whey permeate to traverse said lactose line (20); a sequence of process units (21,22,23,24,25, 26a,26b,27) operatively arranged on said permeate flow path (28), in a first section comprising a heating unit (21), a first caustic tank (26a) configured for addition of a base to said whey permeate, and optionally a permeate bypass flow path (29) for bypassing said heating unit; a second section comprising a holding tank (22), a clarifier (23), and optionally a second caustic tank (26b) configured for addition of a base to said holding tank; a third section comprising an acid tank (27) and a concentration unit (24); and a fourth section comprising a lactose precipitation unit (25); at least one pump for pumping concentrated whey permeate through said lactose line (20); and a controller configured for executing a process according to any of the embodiments of the present invention; wherein in said first section a pH-adjustment step (3) and/or a heating step (4) can be executed; in said second section a precipitation step (5) can be executed in said holding tank (22), optionally under addition of base from said optional second caustic tank (26b), and followed by a clarification step (7); in said third section an acid addition step (8) can be executed followed by a lactose concentration step (9); and in said fourth section a lactose precipitation step (10) can be executed thereby yielding solid lactose (33) and mother liquor (34) depleted in lactose.

The lactose line (20) receives concentrated whey permeate from a source of concentrated whey permeate (30) comprising between 10 to 70% dry solids, preferably 10 to 40% dry solids, which can be obtained in any manner as is useful in the art. Accordingly, the source is not considered limiting on the present invention. The concentrated whey permeate is advantageously depleted in riboflavin.

The lactose line (20) comprises a permeate flow path (28) configured for permitting whey permeate to traverse the lactose line (20) and the sequence of process units (21,22,23,24,25,26a,26b,27) operatively arranged on the permeate flow path (28). To this purpose the permeate flow path connects the process units in a sequence e.g. by the use of pipes or conduits. Since whey permeate is a liquid, it is advantageously pumped through the lactose line (20) from its entry point as concentrated whey permeate (30) (crossing point of stippled line located between items (30) and (28) on Figure 2) and to its exit point (where it exits as mother liquor (34), Figure 2) using at least one pump. The at least one pump will be installed in the permeate flow path (28) according to the specific requirements of the lactose line (20) of the invention. In a preferred embodiment a plurality of pumps are installed in the permeate flow path (28) between the below described sections to assure efficient flow of whey permeate through the lactose line (20).

The sequence of process units (21,22,23,24,25,26a,26b,27) are operatively arranged on the permeate flow path (28) in a manner which is usefully described as a sequence of consecutive sections. In a section matter may enter or exit the permeate flow path (28), without the overall flow direction of the whey permeate thereby being changed. The sections detailed herein are useful in describing the process flow of the whey permeate through the lactose line (20) in that each section will comprise no more than one process unit adding matter and one process unit removing matter from the lactose line (20), however while useful for describing the lactose line (20), this subdivision into sections is otherwise arbitrary and does not influence the execution of the processes of the invention in the lactose line (20).

A controller configured for executing a process according to any of the embodiments of the present invention is further comprised in the lactose line (20).

In this sense a first section can be defined which comprises a heating unit (21), a first caustic tank (26a) configured for addition of a base to the whey permeate, and optionally a permeate bypass flow path (29) for bypassing the heating unit. In this first section a pH-adjustment step (3) and/or a heating step (4) can be executed. In Figure 2 the order of the first caustic tank (26a) and the heating unit (21) is shown with the caustic tank first, however the position of these two process units can be switched without influencing the execution of the process steps (3,4). The heating unit (21) is located in the permeate flow path (28) whereas the first caustic tank (26a) attaches to the permeate flow path (28), e.g. by a valve and an auxiliary pump for pumping caustic from the tank to the permeate flow path. The heating unit is preferably a heat exchanger as is known in the art of continuous whey permeate heating, but may also be a traditional batch boiler. An optional permeate bypass flow path (29) can be included for bypassing the heating unit (21). In operation when heat is not needed, the heating unit (21) can remain switched off permitting the whey permeate to pass the heating unit without being heated, however as the heating unit (21) normally will present a considerable flow restriction, it is advantageous to bypass this unit, when it is not used, by means of the permeate bypass flow path (29).

Further (Figure 2), a second section comprising a holding tank (22), a clarifier (23), and optionally a second caustic tank (26b) configured for addition of a base to the holding tank, can be defined as detailed above. In this second section a precipitation step (5) can be executed in the holding tank (22), optionally under addition of base from the optional second caustic tank (26b), which is followed by a clarification step (7) in the clarifier (23). Clarifiers as detailed above are preferred, however alternative isolation means such as filters or mass centrifuges can be applied in the process as well. The advantage of the clarifier (23), however, is the ability to separate calcium phosphates, CaP, (31) from the whey permeates during continuous operation. The holding tank (22) is located in the permeate flow path (28) whereas the second caustic tank (26a) attaches to the holding tank (22), e.g. by a valve and an auxiliary pump for pumping caustic from the tank to the permeate flow path.

Further (Figure 2), a third section comprising an acid tank (27) and a concentration unit (24) can be defined as detailed above. In this third section an acid addition step (8) can be executed followed by a lactose concentration step (9). The concentration unit (24) is located in the permeate flow path (28) whereas the acid tank (27) attaches to the permeate flow path (28), e.g. by a valve and an auxiliary pump for pumping acid from the tank to the permeate flow path. Suitable concentration units (24) can be evaporation units for evaporating water (32) from the whey permeate, but also nano- or ultrafiltration can suitably be employed as is known in the art of concentrating whey permeates.

Finally (Figure 2), a fourth section comprising a lactose precipitation unit (25) where, in this section, a lactose precipitation step (10) can be executed thereby yielding solid lactose (33) and mother liquor (34) depleted in lactose. In general, the skilled person will know how to precipitate lactose from a solution of concentrated whey permeate and the present invention is not limited by the manner in which precipitation of lactose is executed.

### EXAMPLES - Ash results in crystallized lactose powder:

### Prior Art - Comparative:

Crystallization tank without clarifier and acid: 0% of a 67% lye, 40 min at 75C and no acid gave an ash result of 0,29% and 0,24% measured as sulfated ash

### Present Disclosure:

Crystallization tank with clarifier and acid: 0,075% of a 67% lye to a 20% DM whey permeate, 60 min at 75C, with clarifier and 0,1% of a 37% acid gave an ash result of 0,10% and 0,11% measured as sulfated ash.

When mother liquor of this was purified and processed again at 0% lye, 60 min at 75C with clarifier and 0,1% of a 37% acid then this gave an ash result of 0,12% and 0,12% measured as sulfated ash.

Using clarifier and acid gave a consistent and very good result measured on ash content in the final lactose powder.

### CLOSING COMMENTS

The term "comprising" as used in the claims does not exclude other elements or steps. The term "a" or "an" as used in the claims does not exclude a plurality. And although the present invention has been described in detail for purpose of illustration, it is understood that such detail is solely for that purpose, and variations can be made therein by those skilled in the art without departing from the scope of the invention.

## Claims

1. Process for producing lactose from concentrated whey permeate comprising between 10 to 70% dry solids, said dry solids comprising calcium phosphate, said process comprising the steps of:
ii. Forming precipitates of calcium phosphate in said whey permeate in a precipitation step (5), a fraction of said precipitates having a size larger than a centrifugal clarifier cut-off value;
iii. Clarifying the whey permeate of step ii. in a centrifugal clarification step (7) to remove said fraction of said precipitates having a size larger than said centrifugal clarifier cut-off value to obtain a calcium phosphates reduced whey permeate;
iv. Adding an acid to the calcium phosphates reduced whey permeate of step iii. in an acid addition step (8) to dissolve any remaining precipitates of calcium phosphate, thereby obtaining a precipitate free whey permeate;
vi. Precipitating lactose from said precipitate free whey permeate of step iv. in a lactose precipitation step (10).

2. The process of claim 1 further comprising the step of:
i. Independently, adjusting the pH of the concentrated whey permeate to between pH 6.0 to pH 8.5 in a pH-adjustment step (3) and/or heating the whey permeate to between 60°C to 90°C in a heating step (4) prior to forming said precipitates in step ii.

3. The process of either claim 1 or claim 2 further comprising the step of:
v. Concentrating the precipitate free whey permeate of step iv. in a lactose concentration step (9) to obtain a high lactose content whey permeate prior to precipitating lactose in said lactose precipitation step (10).

4. The process according to any of the claims 1 to 3 wherein, when step v. is present, said concentrated whey permeate preferably comprises between 10 to 40% dry solids, most preferably 20% dry solids, and when step v. is absent, said concentrated whey permeate preferably comprises between 45 to 70% dry solids, most preferably 60% dry solids.

5. The process according to any of the claims 1 to 4 wherein when step i. is performed, said precipitation step (5) is executed for at least 30 minutes.

6. The process according to any of the claims 1 to 5, said concentrated whey permeate comprising between 10 to 70% dry solids, preferably 10 to 40% dry solids, said process comprising the steps of:
i. Independently, adjusting the pH of the concentrated whey permeate to between pH 6.0 to pH 8.5 in a pH-adjustment step (3) and/or heating the whey permeate to between 60°C to 90°C in a heating step (4);
ii. Holding the concentrated whey permeate of step i. for a retention time of at least 30 minutes in a precipitation step (5) in order to allow precipitates of calcium phosphates having a size larger than a centrifugal clarifier cut-off value to form in the whey permeate;
iii. Clarifying the whey permeate of step ii. in a centrifugal clarification step (7) to remove precipitates of calcium phosphates with sizes above said centrifugal clarifier cut-off value to obtain a calcium phosphates reduced whey permeate;
iv. Adding an acid to the calcium phosphates reduced whey permeate of step iii. in an acid addition step (8) to dissolve precipitates of calcium phosphate smaller than said clarifier cut-off value, thereby obtaining a precipitate free whey permeate;
v. Concentrating the precipitate free whey permeate of step iv. in a lactose concentration step (9) to obtain a high lactose content whey permeate; and
vi. Precipitating lactose from said high lactose content whey permeate of step v. in a lactose precipitation step (10).

7. The process of according to any of the claims 2 to 6 comprising both a pH-adjustment step (3) and a heating step (4).

8. The process of according to any of the claims 1 to 7 wherein step ii. or, when present, step i. is preceded by a permeate concentration step (2) and optionally, a riboflavin removal step.

9. The process according to any of the claims 1 to 8 comprising addition of a base to maintain the pH during said precipitation step (5) at the pH of the pH-adjustment step (3).

10. The process according to any of the claims 1 to 9 wherein said centrifugal clarifier cut-off value is 1 µm.

11. The process according to any of the claims 1 to 10 comprising adjusting pH down by 0.05 to 0.5 pH degrees in said acid addition step (8).

12. The process according to any of the claims 3 to 11 wherein said lactose concentration step (9) is concentration by evaporation.

13. The process according to any of the claims 1 to 12 wherein in said precipitation step (5) a base is added to maintain pH at a constant level.

14. A lactose line (20) configured for producing lactose from a source of concentrated whey permeate comprising between 10 to 70% dry solids (30), said lactose line (20) comprising:
- a permeate flow path (28) configured for permitting whey permeate to traverse said lactose line (20);
- a sequence of process units (21,22,23,24,25, 26a,26b,27) operatively arranged on said permeate flow path (28), in:
- a first section comprising a heating unit (21), a first caustic tank (26a) configured for addition of a base to said whey permeate, and optionally a permeate bypass flow path (29) for bypassing said heating unit;
- a second section comprising a holding tank (22), a centrifugal clarifier (23), and optionally a second caustic tank (26b) configured for addition of a base to said holding tank;
- a third section comprising an acid tank (27) and a concentration unit (24); and
- a fourth section comprising a lactose precipitation unit (25);
- at least one pump for pumping concentrated whey permeate through said lactose line (20); and
- a controller configured for executing a process according to any of the claims 1 to 13;
wherein
- said first section is adapted to execute a pH-adjustment step (3) and/or a heating step (4);
- said second section is adapted to execute a precipitation step (5) in said holding tank (22), optionally under addition of base from said optional second caustic tank (26b), and following a centrifugal clarification step (7);
- said third section is adapted to execute an acid addition step (8) followed by a lactose concentration step (9); and
- said fourth section is adapted to execute a lactose precipitation step (10) thereby yielding solid lactose (33) and mother liquor (34) depleted in lactose.

## Patentansprüche

1. Verfahren zur Herstellung von Laktose aus konzentriertem Molkenpermeat, das zwischen 10 und 70 % Trockensubstanz umfasst, wobei die Trockensubstanz Calciumphosphat umfasst, wobei das Verfahren die Schritte umfasst von:
ii. Bilden von Niederschlägen von Calciumphosphat in dem Molkenpermeat in einem Ausfällungsschritt (5), wobei eine Fraktion der Niederschläge eine Größe aufweist, die größer ist als ein Ausschlussgrenzwert der Zentrifugalklärvorrichtung;
iii. Klären des Molkenpermeats von Schritt ii. in einem Zentrifugalklärungsschritt (7), um die Fraktion der Niederschläge zu entfernen, die eine Größe aufweist, die größer ist als der Ausschlussgrenzwert der Zentrifugalklärvorrichtung, um ein calciumphosphatreduziertes Molkenpermeat zu erhalten;
iv. Zugeben einer Säure zu dem calciumphosphatreduzierten Molkenpermeat von Schritt iii. in einem Säureadditionsschritt (8), um alle verbleibenden Niederschläge von Calciumphosphat aufzulösen, wodurch ein niederschlagsfreies Molkenpermeat erhalten wird;
vi. Ausfällen von Laktose aus dem niederschlagsfreien Molkenpermeat von Schritt iv. in einem Laktoseausfällungsschritt (10).

2. Verfahren nach Anspruch 1, ferner umfassend den Schritt:
i. unabhängig voneinander, Einstellen des pH-Wertes des konzentrierten Molkenpermeats auf einen Wert zwischen pH 6,0 und pH 8,5 in einem pH-Einstellungsschritt (3) und/oder Erwärmen des Molkenpermeats auf einen Wert zwischen 60 °C und 90 °C in einem Erwärmungsschritt (4) vor der Bildung der Niederschläge in Schritt ii.

3. Verfahren nach Anspruch 1 oder Anspruch 2, ferner umfassend den Schritt:
v. Konzentrieren des niederschlagsfreien Molkenpermeats von Schritt iv. in einem Laktosekonzentrierungsschritt (9), um ein Molkenpermeat mit hohem Laktosegehalt vor dem Ausfällen der Laktose in dem Laktoseausfällungsschritt (10) zu erhalten.

4. Verfahren nach einem der Ansprüche 1 bis 3, wobei, wenn Schritt v. vorhanden ist, das konzentrierte Molkenpermeat vorzugsweise zwischen 10 bis 40 % Trockensubstanz, am meisten bevorzugt 20 % Trockensubstanz umfasst, und wenn Schritt v. fehlt, das konzentrierte Molkenpermeat vorzugsweise zwischen 45 bis 70 % Trockensubstanz, am meisten bevorzugt 60 % Trockensubstanz umfasst.

5. Verfahren nach einem der Ansprüche 1 bis 4, wobei, wenn Schritt i. durchgeführt wird, der Ausfällungsschritt (5) für mindestens 30 Minuten ausgeführt wird.

6. Verfahren nach einem der Ansprüche 1 bis 5, wobei das konzentrierte Molkenpermeat zwischen 10 und 70 % Trockensubstanz, vorzugsweise zwischen 10 und 40 % Trockensubstanz, umfasst, wobei das Verfahren die Schritte umfasst:
i. unabhängig voneinander, Einstellen des pH-Wertes des konzentrierten Molkenpermeats auf einen Wert zwischen pH 6,0 und pH 8,5 in einem pH-Einstellungsschritt (3) und/oder Erwärmen des Molkenpermeats auf einen Wert zwischen 60 °C und 90 °C in einem Erwärmungsschritt (4);
ii. Halten des konzentrierten Molkenpermeats von Schritt i. für eine Verweildauer von mindestens 30 Minuten in einem Ausfällungsschritt (5), um die Bildung von Niederschlägen von Calciumphosphaten mit einer Größe, die größer als ein Ausschlussgrenzwert der Zentrifugalklärvorrichtung ist, im Molkenpermeat zu ermöglichen;
iii. Klären des Molkenpermeats von Schritt ii. in einem Zentrifugalklärungsschritt (7), um die Niederschläge von Calciumphosphaten mit Größen oberhalb des Ausschlussgrenzwertes der Zentrifugalklärvorrichtung zu entfernen, um ein calciumphosphatreduziertes Molkenpermeat zu erhalten;
iv. Zugeben einer Säure zu dem calciumphosphatreduzierten Molkenpermeat von Schritt iii. in einem Säureadditionsschritt (8), um Niederschläge von Calciumphosphat, die kleiner sind als der Ausschlussgrenzwert der Klärvorrichtung, aufzulösen, wodurch ein niederschlagsfreies Molkenpermeat erhalten wird;
v. Konzentrieren des niederschlagsfreien Molkenpermeats von Schritt iv. in einem Laktosekonzentrierungsschritt (9), um ein Molkenpermeat mit hohem Laktosegehalt zu erhalten; und
vi. Ausfällen von Laktose aus dem niederschlagsfreien Molkenpermeat von Schritt iv. in einem Laktoseausfällungsschritt (10).

7. Verfahren nach einem der Ansprüche 2 bis 6, umfassend sowohl einen pH-Einstellungsschritt (3) als auch einen Erwärmungsschritt (4).

8. Verfahren nach einem der Ansprüche 1 bis 7, wobei Schritt ii. oder, wenn vorhanden, Schritt i. ein Permeatkonzentrierungsschritt (2) und gegebenenfalls ein Riboflavin-Entfernungsschritt vorgeschaltet wird.

9. Verfahren nach einem der Ansprüche 1 bis 8, umfassend das Zugeben einer Base, um den pH-Wert während des Ausfällungsschritts (5) auf dem pH-Wert des pH-Einstellungsschritts (3) zu halten.

10. Verfahren nach einem der Ansprüche 1 bis 9, wobei der Ausschlussgrenzwert der Zentrifugalklärvorrichtung 1 µm beträgt.

11. Verfahren nach einem der Ansprüche 1 bis 10, umfassend das Einstellen des pH-Wertes um 0,05 bis 0,5 pH-Stufen nach unten in dem Säureadditionsschritt (8).

12. Verfahren nach einem der Ansprüche 3 bis 11, wobei der Laktosekonzentrierungsschritt (9) eine Konzentrierung durch Verdampfung ist.

13. Verfahren nach einem der Ansprüche 1 bis 12, wobei in dem Ausfällungsschritt (5) eine Base zugegeben wird, um den pH-Wert auf einem konstanten Niveau zu halten.

14. Laktose-Anlage (20), die zur Herstellung von Laktose aus einer Quelle von konzentriertem Molkenpermeat konfiguriert ist, das zwischen 10 und 70% Trockensubstanz (30) umfasst, wobei die Laktose-Anlage (20) umfasst:
- einen Permeatströmungsweg (28), der konfiguriert ist, um zu ermöglichen, dass das Molkenpermeat die Laktose-Anlage (20) durchläuft;
- eine Aneinanderreihung von Prozesseinheiten (21, 22, 23, 24, 25, 26a, 26b, 27), die funktionsmäßig an dem Permeatströmungsweg (28) angeordnet sind, in:
- einem ersten Abschnitt, der eine Heizeinheit (21), einen ersten Laugetank (26a), der zum Hinzufügen einer Base zu dem Molkenpermeat konfiguriert ist, und gegebenenfalls einen Permeat-Umgehungsströmungsweg (29) zum Umgehen der Heizeinheit umfasst;
- einem zweiten Abschnitt, der einen Vorratstank (22), eine Zentrifugalklärvorrichtung (23) und gegebenenfalls einen zweiten Laugetank (26b) umfasst, der zum Hinzufügen einer Base zu dem Vorratstank konfiguriert ist;
- einem dritten Abschnitt, der einen Säuretank (27) und eine Konzentrierungseinheit (24) umfasst; und
- einem vierten Abschnitt, der eine Laktoseausfällungseinheit (25) umfasst;
- mindestens eine Pumpe zum Pumpen von konzentriertem Molkenpermeat durch die Laktose-Anlage (20); und
- eine Steuerungseinheit, die zum Ausführen eines Prozesses nach einem der Ansprüche 1 bis 13 konfiguriert ist;
wobei
- der erste Abschnitt eingerichtet ist, um einen pH-Einstellungsschritt (3) und/oder einen Erwärmungsschritt (4) auszuführen;
- der zweite Abschnitt eingerichtet ist, um einen Ausfällungsschritt (5) in dem Vorratstank (22), gegebenenfalls unter Zugabe von Base aus dem optionalen zweiten Laugetank (26b) und nachfolgend auf einen Zentrifugalklärungsschritt (7) auszuführen;
- der dritte Abschnitt eingerichtet ist, um einen Säureadditionsschritt (8) auszuführen, gefolgt von einem Laktosekonzentrierungsschritt (9); und
- der vierte Abschnitt eingerichtet ist, um einen Laktoseausfällungsschritt (10) auszuführen, wodurch feste Laktose (33) und Mutterlauge (34), die an Laktose abgereichert ist, erhalten werden.

## Revendications

1. Procédé de production de lactose à partir d'un perméat de lactosérum concentré comprenant de 10 à 70 % de matières solides sèches, lesdites matières solides sèches comprenant du phosphate de calcium, ledit procédé comprenant les étapes suivantes :
ii. formation de précipités de phosphate de calcium dans ledit perméat de lactosérum à une étape de précipitation (5), une fraction desdits précipités ayant une taille supérieure à une valeur seuil de clarificateur centrifuge ;
iii. clarification du perméat de lactosérum de l'étape ii. à une étape de clarification (7) pour éliminer ladite fraction desdits précipités ayant une taille supérieure à ladite valeur seuil de clarificateur centrifuge pour obtenir un perméat de lactosérum à teneur réduite en phosphates de calcium ;
iv. ajout d'un acide au perméat de lactosérum à teneur réduite en phosphates de calcium de l'étape iii. à une étape d'ajout d'acide (8) pour dissoudre tous les précipités de phosphate de calcium restants, permettant ainsi d'obtenir un perméat de lactosérum dépourvu de précipité ;
vi. précipitation du lactose dans ledit perméat de lactosérum dépourvu de précipité de l'étape iv. à une étape de précipitation du lactose (10).

2. Procédé selon la revendication 1 comprenant en outre l'étape suivante :
i. indépendamment, ajustement du pH du perméat de lactosérum concentré à un pH entre pH 6,0 et pH 8,5 à une étape d'ajustement du pH (3) et/ou chauffage du perméat de lactosérum entre 60 °C et 90 °C à une étape de chauffage (4) avant de former lesdits précipités à l'étape ii.

3. Procédé selon la revendication 1 ou la revendication 2 comprenant en outre l'étape suivante :
v. concentration du perméat de lactosérum dépourvu de précipité de l'étape iv. à une étape de concentration du lactose (9) pour obtenir un perméat de lactosérum à teneur élevée en lactose avant la précipitation du lactose à ladite étape de précipitation du lactose (10).

4. Procédé selon l'une quelconque des revendications 1 à 3 dans lequel, lorsque l'étape v. est présente, ledit perméat de lactosérum concentré comprend de préférence de 10 à 40 % de matières solides sèches, de manière davantage préférée 20 % de matières solides sèches, et lorsque l'étape v. est absente, ledit perméat de lactosérum concentré comprend de préférence de 45 à 70 % de matières solides sèches, de manière davantage préférée 60 % de matières solides sèches.

5. Procédé selon l'une quelconque des revendications 1 à 4 dans lequel lorsque l'étape i. est réalisée, ladite étape de précipitation (5) est exécutée pendant au moins 30 minutes.

6. Procédé selon l'une quelconque des revendications 1 à 5, ledit perméat de lactosérum concentré comprenant de préférence de 10 à 70 % de matières solides sèches, de préférence 10 à 40 % de matières solides sèches, ledit procédé comprenant les étapes suivantes :
i. indépendamment, ajustement du pH du perméat de lactosérum concentré à un pH entre pH 6,0 et pH 8,5 à une étape d'ajustement du pH (3) et/ou chauffage du perméat de lactosérum entre 60 °C et 90 °C à une étape de chauffage (4) ;
ii. maintien du perméat de lactosérum concentré de l'étape i. pendant un temps de rétention d'au moins 30 minutes à une étape de précipitation (5) de manière à permettre aux précipités de phosphates de calcium ayant une taille supérieure à une valeur seuil de clarificateur centrifuge de se former dans le perméat de lactosérum ;
iii. clarification du perméat de lactosérum de l'étape ii. à une étape de clarification centrifuge (7) pour éliminer les précipités de phosphates de calcium avec des tailles supérieures à ladite valeur seuil de clarificateur centrifuge pour obtenir un perméat de lactosérum à teneur réduite en phosphates de calcium ;
iv. ajout d'un acide au perméat de lactosérum à teneur réduite en phosphates de calcium de l'étape iii. à une étape d'ajout d'acide (8) pour dissoudre les précipités de phosphates de calcium plus petits que ladite valeur seuil de clarificateur, permettant ainsi d'obtenir un perméat de lactosérum dépourvu de précipité ;
v. concentration du perméat de lactosérum dépourvu de précipité de l'étape iv. à une étape de concentration du lactose (9) pour obtenir un perméat de lactosérum à teneur élevée en lactose ; et
vi. précipitation du lactose dans ledit perméat de lactosérum à teneur élevée en lactose de l'étape v. à une étape de précipitation du lactose (10).

7. Procédé selon l'une quelconque des revendications 2 à 6 comprenant à la fois une étape d'ajustement du pH (3) et une étape de chauffage (4).

8. Procédé selon l'une quelconque des revendications 1 à 7 dans lequel l'étape ii. ou, le cas échéant, l'étape i. est précédée d'une étape de concentration du perméat (2) et facultativement d'une étape d'élimination de la riboflavine.

9. Procédé selon l'une quelconque des revendications 1 à 8 comprenant l'ajout d'une base pour maintenir le pH durant ladite étape de précipitation (5) au niveau du pH de l'étape d'ajustement du pH (3).

10. Procédé selon l'une quelconque des revendications 1 à 9 dans lequel ladite valeur seuil de clarificateur centrifuge est 1 µm.

11. Procédé selon l'une quelconque des revendications 1 à 10 comprenant l'ajustement du pH à la baisse par 0,05 à 0,5 degré de pH à ladite étape d'ajout d'acide (8).

12. Procédé selon l'une quelconque des revendications 3 à 11 dans lequel ladite étape de concentration du lactose (9) est la concentration par évaporation.

13. Procédé selon l'une quelconque des revendications 1 à 12 dans lequel à ladite étape de précipitation (5) une base est ajoutée pour maintenir le pH à un niveau constant.

14. Ligne de lactose (20) configurée pour produire du lactose à partir d'une source de perméat de lactosérum concentré comprenant entre 10 et 70 % de matières solides sèches (30), ladite ligne de lactose (20) comprenant :
- une trajectoire d'écoulement de perméat (28) configurée pour permettre au perméat de lactosérum de traverser ladite ligne de lactose (20) ;
- une séquence d'unités de traitement (21, 22, 23, 24, 25, 26a, 26b, 27) fonctionnellement disposées sur ladite trajectoire d'écoulement de perméat (28), en :
- une première section comprenant une unité de chauffage (21), une première cuve caustique (26a) configurée pour l'ajout d'une base au dit perméat de lactosérum, et facultativement une trajectoire d'écoulement de perméat en dérivation (29) pour court-circuiter ladite unité de chauffage ;
- une deuxième section comprenant une cuve de rétention (22), un clarificateur centrifuge (23), et facultativement une seconde cuve caustique (26b) configurée pour l'ajout d'une base à ladite cuve de rétention ;
- une troisième section comprenant une cuve d'acide (27) et une unité de concentration (24) ; et
- une quatrième section comprenant une unité de précipitation du lactose (25) ;
- au moins une pompe pour pomper le perméat de lactosérum concentré à travers ladite ligne de lactose (20) ; et
- un dispositif de commande configuré pour exécuter un procédé selon l'une quelconque des revendications 1 à 13 ;
dans lequel
- ladite première section est adaptée à exécuter une étape d'ajustement du pH (3) et/ou une étape de chauffage (4) ;
- ladite deuxième section est adaptée à exécuter une étape de précipitation (5) dans ladite cuve de rétention (22), facultativement sous ajout de base de ladite seconde cuve caustique facultative (26b), et après une étape de clarification centrifuge (7) ;
- ladite troisième section est adaptée à exécuter une étape d'ajout d'acide (8) suivie par une étape de concentration de lactose (9) ; et
- ladite quatrième section est adaptée à exécuter une étape de précipitation de lactose (10) pour ainsi donner du lactose solide (33) et de la liqueur mère (34) appauvrie en lactose.
